# EUROPÄISCHE PATENTSCHRIFT

(11) **EP 1 915 716 B1**
(45) Veröffentlichungstag und Bekanntmachung des Hinweises auf die Patenterteilung: **23.05.2018**
(21) Anmeldenummer: 06779944.5
(22) Anmeldetag: 28.07.2006
(51) Int. Cl.: G01G 19/414, G06F 19/00

(54) **ELEKTRONISCHE WAAGE UND VERFAHREN ZUR KONTROLLE DER SPEISEEINAHME BZW. DER VERSPEISTEN KALORIENMENGEN**
ELECTRONIC SCALES AND METHOD FOR CONTROLLING FOOD INTAKE AND/OR THE AMOUNT OF CALORIES CONSUMED
BALANCE ELECTRONIQUE ET PROCEDE POUR CONTROLER LA PRISE ALIMENTAIRE OU LES QUANTITES DE CALORIES CONSOMMEES

(30) Priorität: 16.08.2005 CH 13392005
(43) Veröffentlichungstag der Anmeldung: 30.04.2008
(73) Patentinhaber: Rump, Björn S., 1253 Vandoeuvres (CH)
(72) Erfinder: Rump, Björn S., 1253 Vandoeuvres (CH)
(74) Vertreter: Nuss, Laurent
(86) Internationale Anmeldenummer: PCT/IB2006/002164
(87) Internationale Veröffentlichungsnummer: WO 2007/020501

(56) Entgegenhaltungen:
- WO-A2-02/25228
- DE-A1- 4 125 210
- DE-U1- 29 924 679
- GB-A- 2 317 961
- US-A1- 2003 159 857

## Beschreibung

Die vorliegende Erfindung betrifft das Gebiet der Diätkontrolle, insbesondere der hierzu zur Verfügung stehenden Geräten und Vorrichtungen.

Die Erfindung schliesst eine elektronische Waage zur effizienten Kontrolle der verspeisten Kalorienmengen, sowie ein Verfahren zur Kontrolle der Speiseeinahme eines Individuums, ein.

Es sind zahlreiche Programme und Methoden zur Verringerung des Körpergewichts bekannt, sei es nun aus kosmetischen, wohlbefindlichen oder therapeutischen Gründen.

Viele dieser bekannten Programme und Methoden erlauben es in der Tat, während ihrer aktiven Anwendungsphase das Körpergewicht zu verringern. Dies geschieht in den meisten Fällen durch eine Verminderung der Kalorien-Einnahme.

Die Kontrolle einer solchen Diät beruht auf dem detaillierten Wiegen oder Abschätzen des Gewichtes aller Speisen während und zwischen den Mahlzeiten, der Berechnung der entsprechenden Kalorien mittels einer Kalorientabelle und der Eintragung aller errechneten Beträge in eine Tages-, Wochen- und Monatstabelle.

Die Praxis zeigt jedoch, dass die Einhaltung dieser systematischen Kontrolle bei den meisten Diätkandidaten nur kurzfristig ist oder sich im besten Falle nur über eine mehrmonatige Periode erstreckt.

Die Erklärung hierzu liegt zum grossen Teil bei der Mühsamkeit der vielzähligen Verfahrensschritte, des benötigten Zeitaufwands, der Abwesenheit einer systematischen und automatisierten Kontrollprozedur, der effizienten Änderung der Essgewohnheiten, des im Gebrauch unpraktischen Zubehörs und der Störung der Bequemlichkeit des Kandidaten.

Aus der GB-A-2 317 961 ist eine elektronische Waage gemäss Oberbegriff des Patentanspruchs 1 bekannt, sowie ein entsprechendes Benutzungsprogramm.

Hier werden zur Bestimmung der Kalorienmenge Speisen und Getränke im Anschluss aneinander gewogen. Ein bestimmter Diätplan an sich wird in dieser Vorveröffentlichung nicht offenbart.

Es liegt der Erfindung die Aufgabe zu Grunde, eine Waage des vorgenannten Typs so zu verbessern, dass deren Benutzung einfacher und effizienter ist und durch Energieeinsparung auch verlängert werden kann.

Diese Aufgabe wird durch die Merkmale des kennzeichnenden Teils des Anspruchs 1 erfindungsgemäss gelöst.

Ferner liegt der Erfindung auch die weitere Aufgabe zu Grunde ein Verfahren zum effizienten Gewichtsverlust und dessen Beibehaltung unter Verwendung der vorgenannten Waage vorzuschlagen.

Diese weitere Aufgabe wird mit dem Verfahren gemäss Anspruch 10 gelöst.

In der folgenden Beschreibung wird nun die Erfindung an Hand eines Ausführungsbeispiels näher erläutert, dies im Zusammenhang mit den anliegenden schematischen Figuren bei denen:
Figur 1A eine schematische Draufsicht einer Waage gemäss der Erfindung in Gebrauchslage zeigt;
Figur 1B eine seitliche Ansicht der Waage der Figur 1A in gefalteten Zustand zeigt, und

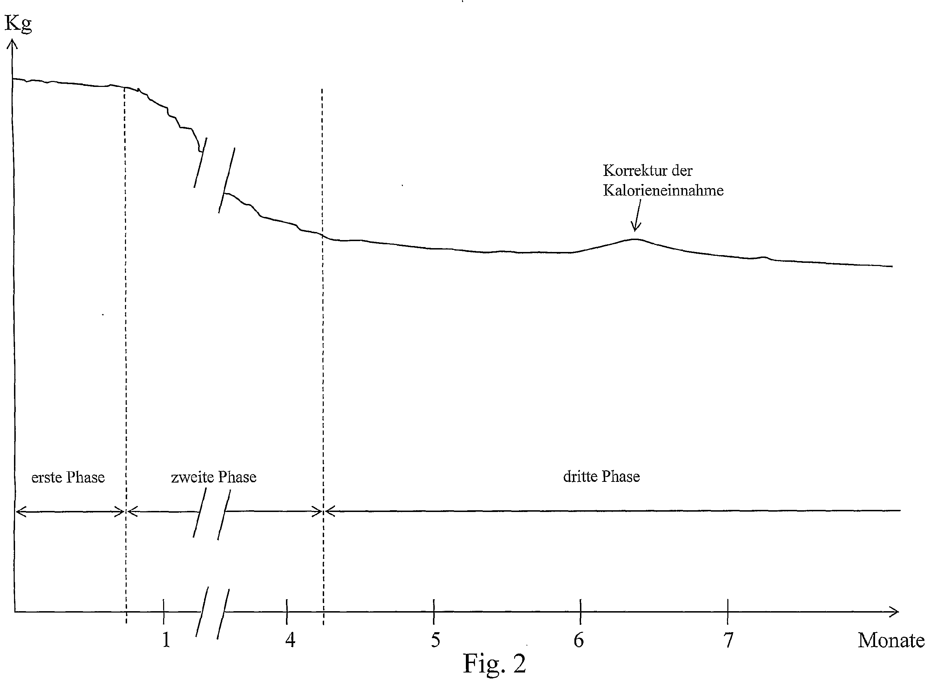

Figur 2 ein schematisches Zeitdiagramm ist, welches, beispielsweise die zeitliche Änderung des Körpergewichts einer Person zeigt, welche das erfindungsgemässe Verfahren anwendet.

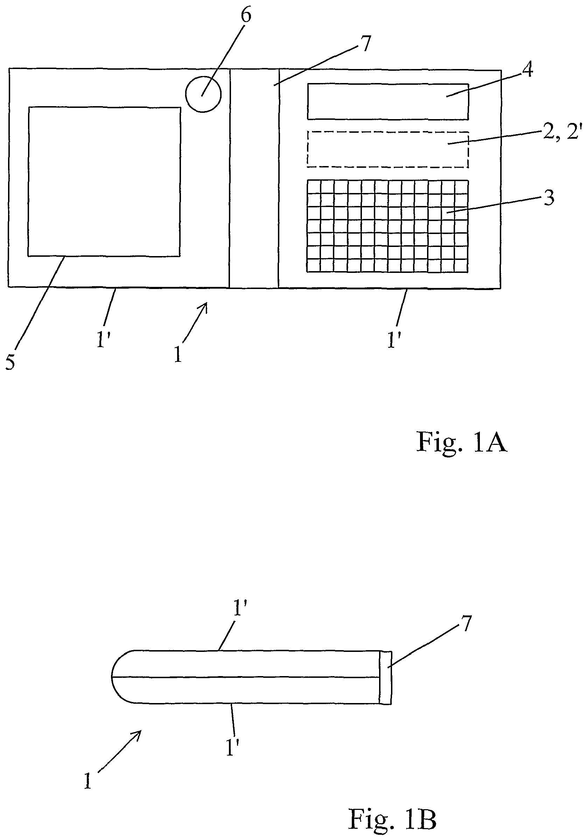

Wie dies aus den anliegenden Figuren 1A und 1B hervorgeht, ist die Waage 1 mit wenigstens einem Gewichts- oder Kraftsensor, einer Datenverarbeitungseinheit 2 mit Speichermitteln 2', einer Steuer- und Eingabetastatur 3 und einer Anzeigevorrichtung 4 ausgestattet.

Erfindungsgemäss ist vorgesehen, dass sie eine zum Aufnehmen eines Speisetellers oder ähnlichen Behälters angepasste und mit dem oder den Gewichts- oder Kraftsensor(en) funktionell verbundene aktive Auflagefläche 5 aufweist, dass die Datenverarbeitungseinheit 2 mit einem Programm ausgestattet ist welches für jede Speise, in Form von flüssiger und/oder fester Nahrung, auf der Basis der gewogenen Speisemenge und des gespeicherten oder manuell eingegebenen gewichtsbezogenen Kaloriengehalts dieser Speise, den Kalorienäquivalenzwert berechnet, letzteren mit den Kalorien-äquivalenzwerten der anderen Speisen eines Mahls summiert und einen Gesamtwert der Kalorien der täglich eingenommenen Speisen errechnet und diesen Wert speichert, anzeigt und/oder ausdruckt und dass die Speichermittel 2' dazu geeignet sind vorprogrammierte Sollwerte zu enthalten, welche aus den Dateneingaben und Messungen einer Kalibrierungsperiode errechnet werden und als Vergleichs- oder Referenzwerte während der Kalorienkontrollperiode dienen.

Der Benutzer verfügt somit über eine kompakte und leicht handhabende Vorrichtung, die ihm die genaue Kontrolle seiner Diät wesentlich erleichtert.

Damit ein fehlerfreies und präzises Funktionieren der Waage 1 gesichert werden kann, ist vorgesehen, dass die Auflagefläche 5 und die ihr zugeordneten Gewichts- oder Kraftsensoren so ausgelegt sind, dass Teller verschiedener Grössen und Formen genau gewogen werden können und dass das Programm eine Routine beinhaltet, welche während jeder Gewichtsmessphase überprüft, dass jeder Gewichts- oder Kraftsensor belastet ist und ein zum effektiven Messresultat beitragendes korrektes Messsignal liefert.

Um im Zusatz zu den Nahrungen in Feststoffform auch die zur Mahlzeiten eingenommenen Flüssigstoffen und Getränke auswerten zu können, beinhaltet die Waage 1 mindestens eine zweite aktive Auflagefläche 6, welche separat von der ersten Auflagefläche 5 ist, mit eigenem(n) Gewichts- oder Kraftsensor(en) ausgestattet und hiermit funktionell verbunden ist und zur Aufnahme von Trinkgefässen oder - behältern, wie z. B. Gläser oder Tassen, geeignet ist, wobei mindestens ein zweites Messignal hiervon geliefert wird.

Damit die Waage 1 in der Praxis leicht einzusetzen, mitzunehmen und aufzuräumen ist, ist ihr Gesamtaufbau im wesentlichen flächig und dünn (und gleicht zum Beispiel dem eines Telleruntersetzers), mit einer Auflagefläche 5 für den Teller und einer Auflagefläche 6 für ein Trinkgefäss, wobei die Programmierungs- und Steuerungstastatur 3, sowie die z. B. aus Flüssigkristalldioden bestehende Anzeigevorrichtung 4, seitlich verschoben angeordnet sind, und somit auch bei Vorhandensein eines Tellers und/oder eines Trinkgefässes, ersichtlich bzw. erreichbar sind.

Wie es die Figuren 1A und 1B zeigen, kann die Waage zum Beispiel aus zwei flachförmigen Hüllen 1' bestehen, welche über ein Scharnierteil 7 gelenkig miteinander verbunden sind, wobei die Waage somit bei Nichtgebrauch und/oder zum Transport zusammengefaltet werden kann.

Die Hüllen 1', die die funktionellen Komponenten der Waage 1 umgeben und die äusserliche Form ihres Gesamtaufbaus bestimmen, bestehen vorzugsweise aus einem steifen Werkstoff, wie Edelstahlblech oder Plastik, der für Reinigung nach dem Nahrungskontakt geeignet ist. Erfindungsgemäß beinhaltet die Waage auch Blockiermittel, welche es erlauben, die Auflagefläche 5 für den Teller, und gegebenenfalls die Auflagefläche 6 für das Trinkgefäss, ausserhalb einer Mess- und Verrechnungsphase, in einer gegebenen Stellung starr zu verriegeln und/oder jegliche Datenübergabe als Messignale an die Datenverarbeitungsenheit 2 blockiert und verhindert, wobei somit die zum Speisen und Trinken erforderlichen Bewegungen sicher und störungsfrei ausgeführt werden können.

Gemäss einem vorteilhaften Merkmal der Erfindung, enthalten die Speichermittel 2' eine programmierbare, ergänzbare und abfragbare Kalorienäquivalenztabelle, welche durch Dateneingabe über die Tastatur 3 ergänzt und abgefragt werden kann, wobei das Abfragen durch Eingabe nur eines Teils der Buchstaben des Namens der betroffenen Speise/Nahrung erfolgen kann und der jeweils abgefragte Wert automatisch von der Datenverarbeitungseinheit 2 als Multiplikationsfaktor für die Berechnung des Kalorienwertes einer gewogenen Speise/Nahrung verwendet wird.

Zum Beispiel kann vorgesehen werden, dass wenn der Benutzer die Buchstaben "BR" eingibt, die Anzeigemittel 4 das Wort "BROT" und andere Wörter, die mit "BR" beginnen angibt, wobei das zutreffende Wort dann nur validiert werden muss.

Weiterhin kann vorgesehen sein, dass die abfragbare Kalorienäquivalenztabelle, für Personen welche an einer Krankheit leiden und dadurch spezifisch täglich mindest oder maximal Werte eines Elementes oder Moleküls einnehmen sollen, den spezifischen Gehalt der kritischen Anteile der vorgegebenen Speiseliste enthält und dessen Tagestotal ebenfalls berechnet wird.

Diese kritischen Anteile können durch die Tastatur eingegeben werden (zum Beispiel Apfelsaft x g Glukosid/100 g Flüssigkeit im Falle einer Person, die an akuter Diabetes leidet).

Es ist auch vorgesehen, dass das Programm, auf entsprechende Steuerung durch die Tastatur 3 hin, das Leergewicht des Tellers und/oder des Trinkgefässes misst und speichert, d. h. die Tara bestimmt, und danach den netto Kalorienwert jeder aufgelegten Speise/Nahrung einer Mahlzeit, den gesamt Kalorienwert jeder Mahlzeit und den kumulierten Kalorienwert der verschiedenen Mahlzeiten eines Tages berechnet und speichert, wobei gegebenenfalls auch die plus/minus Differenz zu einem vorab eingegebenen oder vorgerechneten täglichen Kaloriensollwert berechnet und angezeigt wird.

Die gespeicherten Werte, gegebenenfalls ergänzt durch Datum- und/oder Zeitangabe und/oder durch manuell über die Tastatur 3 eingegebene Daten, können von entsprechenden, integrierten oder separaten verbundenen Mitteln auf einem Datenträger, wie z. B. ein Blatt Papier, eine elektronische Datenkarte, ein Festkörperspeicher oder ähnlich, übermittelt werden.

Ferner kann die Waage ebenfalls Mittel zur visuellen oder hörbaren Wiedergabe von Meldungen, insbesondere von Resultatmeldungen, Kommentaren, Warnungen, Abweichungen vom gespeicherten Diätprogramm und ähnlich, aufweisen.

Die Erfindung bezieht sich auch auf ein Verfahren zur Kontrolle der Speiseeinahme eines Individuums im Rahmen einer Diät und unter Verwendung einer elektronischen Waage wie oben beschrieben. Dieses Verfahren besteht zeitlich aus drei aufeinanderfolgenden Phasen, nämlich einer ersten zeitlich begrenzten Kalibrierungsperiode, einer darauf folgenden effektiven Diätperiode und schliesslich einer langzeitlichen Stabilisierungsperiode, wobei während aller drei Perioden die täglichen Kalorienäquivalenzwerte der eingenommenen Speisen mittels der elektronischen Waage 1 berechnet werden, durch Wiegen der einzelnen Speisen jeder Mahlzeit, Errechnen der entsprechenden Kalorien und Summieren letzterer für jeden Tag dieser drei Perioden, wobei in der dritten Periode oder Stabilisierungsperiode die Effizienz, bzw. die Richtigkeit der täglich eingenommenen Kalorienmenge durch regelmässiges Wiegen des Körpergewichtes kontrolliert, und falls nötig empirisch zur Beibehaltung des gewünschten Körpergewichtes korrigiert wird.

Gemäss der Erfindung dienen die Werte bezüglich täglicher Kalorieneinnahme des Individuums während der ersten Phase als Basis zur Berechnung von Referenzwerten für die zweite und dritte Phase, wobei in der zweiten und dritten Phase eine Abweichung des effektiven täglichen Kalorieneinnahmewertes von dem vorberechneten entsprechenden Referenzwert angegeben, angezeigt und/oder gemeldet wird.

Vorteilhafterweise stellt der Referenzwert der zweiten Phase 0,75 bis 0,90 des täglichen Mittelwerts der ersten Phase und der Referenzwert der dritten Phase 0,95 bis 1,15 des täglichen Mittelwerts der zweiten Phase dar, wobei der Referenzwert der dritten Phase gegebenenfalls, im Zusammenhang mit einer anhaltenden Gewichtsänderung des Individuums, empirisch einstellbar ist.

Die erste Phase dient zur Bestimmung der mittleren täglichen Kalorieneinahme vor der Diät. Die zweite Phase endet mit dem Erreichen eines gewünschten Gewichts des Individuums. Die dritte Phase dient dazu, die neue reduzierte Speiseeinahme zu einer eigentlichen Essgewohnheit werden zu lassen.

Es kann weiterhin vorgesehen werden, dass für spezifische Diätprogramme neben dem totalen Kalorienwert der aufgenommenen Nahrung, durch Erweiterung der gespeicherten Kalorienwerten der einzelnen Nahrungsmittel, durch zusätzliche Speicherung des Gehaltes an Eiweiss oder Proteine, Kohlehydrate, Fett oder andere aus medizinischen Gründen zu begrenzende Nahrungsstoffe gemessen und dadurch in den gewünschten Grenzen gehalten werden können.

Die verschiedenen Gegenstände und Merkmale der Erfindung werden nun nachstehend an Hand eines Beispiels näher erläutet.

Die erfinderische Lösung besteht aus drei Teilen, nämlich aus einem Verfahren (oder Methode), einem Gerät (elektronische Waage) und einem Steuerungs-, Kontroll- und Kommunikationsprogramm, welches vom Gerät ausgeführt und zu dessen Betrieb genutzt wird.

Die Methode an sich besteht darin, zuerst für ca. zwei Wochen die tägliche Nahrungsmittelmenge der Person, welche ihr Körpergewicht dauerhaft verringern will, den mittleren täglichen Kalorienwert, auf der Basis der während der Mahlzeiten und Zwischenmahlzeiten verzehrten Speisen und Getränke zu bestimmen.

Sobald dieser Kalorienwert bestimmt ist, wird er um ca. 10 - 25% verringert und die betreffende Person legt für sich fest, sich auf die entsprechende Nahrungsmittelmenge zu beschränken. Das Einhalten dieser Diät ist ohne grosse Einschränkungen d.h. Hungergefühle möglich. Hingegen ist es unmöglich diese Beschränkung ohne korrekte Bestimmung und Kenntnisnahme der verzehrten Kalorien einzuhalten.

Das Körpergewicht der Person wird wöchentlich gewogen und auf einer Grafik aufgezeichnet. Der sich auf der Graphik ersichtliche Trend ist für die Person eine sehr starke Motivation die Diätdisziplin weiterhin aufrecht zu erhalten.

Ist nach einigen Monaten das erwünschte Körpergewicht erreicht, beginnt die wichtigste Phase des Programms. Die tägliche Kalorienmenge wird um ca. 5% erhöht und deren Einnahme während Monaten kontrolliert und beibehalten. Durch das wöchentliche Wiegen des Gewichts wird dessen weitere Entwicklung bestimmt. Ändert sich in dieser Zeit das Gewicht, ist es leicht möglich -da man den täglichen Verzehr kennt- diesen empirisch nach oben oder nach unten anzupassen. Das Endziel ist, Essgewohnheiten zu erlernen, welche die tägliche Kontrolle des Verzehrs überflüssig machen. Die Erfahrung vieler Diätprogramme hat gezeigt, dass die gewünschte Gewichtsabnahme durchaus möglich ist, dass aber in fast allen Fällen der gefürchtete sogenannte Yoyo-effekt eintritt, d.h. dass das Gewicht der Person bald wieder den Ausgangswert erreicht. Die vorliegende Methode soll der Person, welche abnehmen will, den Weg zur dauerhaften Gewichtsverringerung durch Angewöhnung anderer Essgewohnheiten so leicht als möglich machen.

Für die Erleichterung der Einhaltung der beschriebenen Methode wird während der Mahlzeiten die Waage 1 gemäss der Erfindung unter den Speiseteller gestellt.

Diese Waage ist so zu gestalten, dass sie unter einen Speiseteller passt und dass die Gewichtsanzeige durch den in die Waage 1 eingebauten Rechner 2 gespeichert, mit dem Kalorienfaktor der Speise multipliziert und die so bestimmte Kalorienmenge zum Tagestotal addiert und angezeigt wird. Dieser vereinfachte Vorgang erleichtert die erforderliche Einnahmekontrolle wesentlich, erfordert weniger Verhaltensdisziplin und kann, da sie mit wenig Aufwand durchgeführt werden kann, über längere Zeit angewendet werden, während welcher es möglich ist, wie erwähnt die Essgewohnheiten dauerhaft zu ändern.

Die Waage 1 besitzt wenigstens eine Auflagefläche 5, die dem gängigen Durchmesser der Speiseteller entspricht, ist möglichst leicht gebaut und zeigt das Wiegeresultat in "g" in wenigen Sekunden auf einem Flüssigkristalldiodenbildschirm 4 an. Durch Auflegen eines Tellers wird die Waage eingeschaltet und auf Null gestellt (Tara). Nach der Beladung des Tellers mit einer Speise tippt der Benützer mit einer numerischen Klaviatur 3 (0 - 9) den ihm bekannten Kaloriengehalt der auf den Teller geladenen Speise ein und betätigt darauf den "Enter-Knopf'. Auf die vom Fachmann bekannte Art, berechnet der Mikroprozessor der Datenverarbeitungseinheit 2 den Kalorienwert, der auf dem Teller geladenen Speise und zeigt diesen auf dem Bildschirm (Anzeigemittel 4) an. Durch "Enter" wird das Resultat zum Total addiert. Beim Aufladen von weiteren Speisemengen wird der Vorgang entsprechend wiederholt. Vor dem Beginn des Verzehrs wird die Waage blockiert, so dass der Teller auf der Waage liegen bleiben kann und die vorliegenden Speisen zerschnitten und gegessen werden können, ohne dass die Resultate geändert oder beeinflusst werden.

Bei der nächsten Mahlzeit kann die Waage 1 durch einen "Enter" aktiviert werden, ohne dass die vorherige Kalorienzahl (diejenige der letzten Mahlzeit) gelöscht wird. Die folgende Mahlzeit d.h. deren Kalorienwert wird dann in beschriebener Weise bestimmt. Nach der letzten Mahlzeit eines Tages kann, durch entsprechenden Tastendruck, das Tagestotal und, falls gewünscht, auch die Abweichung vom gewünschten Soll angezeigt werden. Diese Anzeige hat einen pädagogischen Wert, sie belohnt die angewendete Disziplin oder warnt bei Überschreitungen. Die durch die Methode bestimmten Kalorienmengen können ohne speziell grosse Überwindung eingehalten werden.

Dem Fachmann ist die praktische Ausführung der Waage 1 und des Programms naheliegend im Bezug auf seine Kenntnisse, die Entwicklung der nötigen Logistik ebenfalls. Es liegt auf der Hand, dass die gewünschte Kontrollfunktion auf verschiedene Arten verwirklicht werden kann, ohne dass vom Prinzip der einfachen Totalisierung der Kalorienmenge abgewichen wird.

Die Vorrichtung kann z. B. durch einen Speicher einer Kalorientabelle ergänzt werden. Nach dem Wiegevorgang können nach bekannter Art die ersten Buchstaben der Speise eingetippt werden. Dieser Vorgang wird beendet, sobald der gesuchte Speisename auf dem Bildschirm erscheint. Auch dieser Vorgang ist dem Fachmann bekannt.

Da die Getränke ebenfalls Kalorien enthalten, kann die Waage zusätzlich mit einer Wagefläche 6 für Gläser oder Tassen ausgerüstet werden, welche das Erfassen der eingeschenkten Flüssigkeitsmenge sinngemäss erlaubt.

Da viele Personen häufig nicht nur zu Hause speisen, ist eine Ausführung der Waage in einer möglichst leichten und flachen Form von Vorteil, welche es erlaubt, das Gerät in ein Restaurant oder zu einer Einladung mitzunehmen und da zu benutzen.

Die Waage 1 kann auch so programmiert werden, dass der Benutzer den anfallenden Kalorienwert schätzen und ihn danach mit dem Gemessenen vergleichen muss. Einem motivierten Benutzer kann diese Eigenschaft erlauben, die Kalorienwerte mit genügender Genauigkeit schätzen zu lernen, was ihm erlaubt mit der Zeit auf die detaillierte Kontrolle zu verzichten.

Das sich in der Waage 1 befindliche Rechen- Steuerungs- und Verwaltungsprogramm kann z. B. folgende Funktionen aufweisen bzw. ausführen:
a. Bestimmen des Auflagegewichtes und dessen Anzeige
b. Aufnahme des manuell eingegebenen Kalorienwertes Multiplikation der beiden ersten Werte und deren Anzeige
c. Speichern von Kalorienäquivalenzwerten und Berechnen nach Eintippen der ersten Buchstaben des Speisenamens (z. B. ka → Kartoffel), des Kalorienwertes der aufgelegten Speisen.
d. Addierung des neuen Kalorienwertes zum vorherigen
e. Anzeige des Tagestotals
f. Berechnung und Anzeige von Werten, welche die Anwendung vereinfachen und dem Benutzer die Kontrolle seiner Disziplin erlauben und ihn zum Einhalten des Programms motivieren, z. B. durch Angabe der Differenz des Tagestotals zum Sollwert.
g. Der Konstrukteur des Gerätes hat die Freiheit, durch einen integrierten oder äusseren Drucker die Resultate und deren Total und eventuell motivierende Bemerkungen dem Benutzer bekannt zu geben.
h. Berechnen und Substraieren der Kalorienmenge der nicht eingenommenen Speisen.

Selbstverständlich ist die Erfindung nicht auf die beschriebenen und dargestellten Ausführungsformen begrenzt. Änderungen, z. B. in den Ausführungsformen der verschiedenen Bestandteile oder Ersetzen durch technische Äquivalente sind, soweit sie im Rahmen des beanspruchten Schutzbegehrens bleiben, jederzeit möglich.

## Patentansprüche

1. Elektronische Waage zur effizienten Kontrolle der verspeisten Kalorienmengen, mit wenigstens einem Gewichts- oder Kraftsensor, einer Datenverarbeitungseinheit mit Speichermitteln, einer Steuer- und Eingabetastatur und einer Anzeigevorrichtung,
wobei diese Waage (1) eine zum Aufnehmen eines Speisetellers oder ähnlichen Behälters angepasste und mit dem oder den Gewichts- oder Kraftsensor(en) funktionell verbundene aktive Auflagefläche (5) aufweist,
wobei die Datenverarbeitungseinheit (2) mit einem Programm ausgestattet ist welches für jede Speise, in Form von flüssiger und/oder fester Nahrung, auf der Basis der gewogenen Speisemenge und des gespeicherten oder manuell eingegebenen gewichtsbezogenen Kaloriengehalts dieser Speise, den Kalorienäquivalenzwert berechnet, letzteren mit den Kalorien-äquivalenzwerten der anderen Speisen eines Mahls summiert und einen Gesamtwert der Kalorien der täglich eingenommenen Speisen errechnet und diesen Wert speichert, anzeigt und/oder ausdruckt,
wobei der Gesamtaufbau der Waage (1) im wesentlichen flächig und dünn ist,
wobei die Speichermittel (2') dazu geeignet sind vorprogrammierte Sollwerte zu enthalten, welche aus den Dateneingaben und Messungen einer Kalibrierungsperiode durch die Datenverarbeitungseinheit (2) errechnet werden und als Vergleichs- oder Referenzwerte während der Kalorienkontrollperiode dienen,
Waage (1) **dadurch gekennzeichnet:**
- **dass** sie mindestens eine zweite aktive Auflagefläche (6) beinhaltet, welche separat von der ersten Auflagefläche (5) ist, mit eigenem(n) Gewichts- oder Kraftsensor(en) ausgestattet und hiermit funktionell verbunden ist und zur Aufnahme von Trinkgefässen oder - behältern, wie z. B. Gläser oder Tassen, geeignet ist, wobei mindestens ein zweites Messsignal hiervon geliefert wird,
- **dass** sie somit eine Auflagefläche (5) für den Teller und eine Auflagefläche (6) für ein Trinkgefäss aufweist,
- **dass** sie Blockiermittel beinhaltet, welche es erlauben, die Auflagefläche (5) für den Teller, und gegebenenfalls die Auflagefläche (6) für das Trinkgefäss, ausserhalb einer Mess- und Verrechnungsphase, in einer gegebenen Stellung starr zu verriegeln und/oder jegliche Datenübergabe als Messsignale an die Datenverarbeitungseinheit (2) blockiert und verhindert.

2. Elektronische Waage nach Anspruch 1, **dadurch gekennzeichnet, dass** die Auflagefläche (5) und die ihr zugeordneten Gewichts- oder Kraftsensoren so ausgelegt sind, dass Teller verschiedener Grössen und Formen genau gewogen werden können und dass das Programm eine Routine beinhaltet, welche während jeder Gewichtsmessphase überprüft, dass jeder Gewichts- oder Kraftsensor belastet ist und ein zum Messresultat beitragendes korrektes Messsignal liefert.

3. Elektronische Waage nach Anspruch 1 oder 2, **dadurch gekennzeichnet dass** die Programmierungs- und Steuerungstastatur (3), sowie die z. B. aus Flüssigkristalldioden bestehende Anzeigevorrichtung (4), seitlich verschoben angeordnet sind, und somit auch bei Vorhandensein eines Tellers und/oder eines Trinkgefässes, ersichtlich bzw. erreichbar sind.

4. Elektronische Waage nach Anspruch 3, **dadurch gekennzeichnet, dass** sie aus zwei flachförmigen Hüllen (1') besteht, welche über ein Scharnierteil (7) gelenkig miteinander verbunden sind, wobei die Waage somit bei Nichtgebrauch und/oder zum Transport zusammengefaltet werden kann.

5. Elektronische Waage nach einem der Ansprüche 1 bis 4, **dadurch gekennzeichnet, dass** die Speichermittel (2') eine programmierbare, ergänzbare und abfragbare Kalorienäquivalenztabelle enthalten, welche durch Dateneingabe über die Tastatur (3) ergänzt und abgefragt werden kann, wobei das Abfragen durch Eingabe nur eines Teils der Buchstaben des Namens der betroffenen Speise/Nahrung erfolgen kann und der jeweils abgefragte Wert automatisch von der Datenverarbeitungseinheit (2) als Multiplikationsfaktor für die Berechnung des Kalorienwerts einer gewogenen Speise/Nahrung verwendet wird.

6. Elektronische Waage nach Anspruch 5, **dadurch gekennzeichnet, dass** die abfragbare Kalorienäquivalenztabelle, für Personen welche an einer Krankheit leiden und dadurch spezifisch täglich mindest oder maximal Werte eines Elementes oder Moleküls einnehmen sollen, den spezifischen Gehalt der kritischen Anteile der vorgegebenen Speiseliste enthält, und dass dessen Tagestotal ebenfalls berechnet wird.

7. Elektronische Waage nach einem der Ansprüche 1 bis 6, **dadurch gekennzeichnet, dass** das Programm, auf entsprechende Steuerung durch die Tastatur (3) hin, das Leergewicht des Tellers und/oder des Trinkgefässes misst und speichert und danach den Kalorienwert jeder aufgelegten Speise/Nahrung einer Mahlzeit, den gesamt Kalorienwert jeder Mahlzeit und den kumulierten Kalorienwert der verschiedenen Mahlzeiten eines Tages berechnet und speichert, wobei gegebenenfalls auch die plus/minus Differenz zu einem vorab eingegebenen oder vorgerechneten täglichen Kaloriensollwert berechnet und angezeigt wird.

8. Elektronische Waage nach einem der Ansprüche 1 bis 7, **dadurch gekennzeichnet, dass** die gespeicherten Werte, gegebenenfalls ergänzt durch Datum- und/oder Zeitangabe und/oder durch manuell über die Tastatur (3) eingegebene Daten, von entsprechenden, integrierten oder separaten verbundenen Mitteln auf einem Datenträger, wie z. B. ein Blatt Papier, eine elektronische Datenkarte, ein Festkörperspeicher oder ähnlich, übermittelt werden.

9. Elektronische Waage nach einem der Ansprüche 1 bis 8, **dadurch gekennzeichnet, dass** sie ebenfalls Mittel zur visuellen oder hörbaren Wiedergabe von Meldungen, insbesondere von Resultatsmeldungen, Kommentaren, Warnungen, Abweichungen vom gespeicherten Diätprogramm und ähnlich, aufweist.

10. Verfahren zur Kontrolle der Speiseeinnahme eines Individuums im Rahmen einer Diät und durch Verwendung einer elektronischen Waage nach einem der Ansprüche 1 bis 9, welche eine erste aktive Auflagefläche (5) zum Aufnehmen eines Tellers oder eines ähnlichen Behälters und mindestens eine zweite separate aktive Auflagefläche (6) zum Aufnehmen eines Trinkgefässes oder -behälters aufweist,
wobei das Verfahren zeitlich aus drei aufeinanderfolgenden Phasen besteht, nämlich einer ersten zeitlich begrenzten Kalibrierungsperiode, einer darauf folgenden effektiven Diätperiode und schliesslich einer langzeitlichen Stabilisierungsperiode,
wobei während aller drei Perioden die täglichen Kalorienäquivalenzwerte der eingenommenen Speisen mittels der elektronischen Waage (1) berechnet werden, durch Wiegen der einzelnen Speisen jeder Mahlzeit, Errechnen der entsprechenden Kalorien und Summieren letzteren für jeden Tag dieser drei Perioden,
wobei die erste Auflagefläche (5) ein erstes Messsignal und die zweite Auflagefläche (6) ein zweites Messsignal liefert, wobei die Waage (2) aus den Dateneingaben und Messungen der Kalibrierungsperiode tägliche Kaloriensollwerte berechnet und speichert, welche als Vergleichs- oder Referenzwerte während den zwei folgenden Perioden dienen,
wobei die Waage (2) während der Mähler als Telleruntersetzer dient, nach Verriegelung in einer gegebenen Stellung und Blockierung jeglicher Datenübergabe als Messsignale an die Datenverarbeitungs- einheit (2),
wobei in der dritten Periode oder Stabilisierungsperiode die Effizienz, bzw. die Richtigkeit der täglich eingenommenen Kalorienmenge durch regelmässiges Wiegen des Körpergewichtes kontrolliert, und falls nötig empirisch zur Beibehaltung des gewünschten Körpergewichtes korrigiert wird.

11. Verfahren nach Anspruch 10, **dadurch gekennzeichnet, dass** die Werte bezüglich täglicher Kalorieneinnahme des Individuums während der ersten Phase als Basis zur Berechnung von Referenzwerten für die zweite und dritte Phase dienen, wobei in der zweiten und dritten Phase eine Abweichung des effektiven täglichen Kalorieneinnahmewertes von dem vorberechneten entsprechenden Referenzwert angegeben, angezeigt und/oder gemeldet wird.

12. Verfahren nach Anspruch 10 oder 11, **dadurch gekennzeichnet, dass** der Referenzwert der zweiten Phase 0,75 bis 0,90 des täglichen Mittelwerts der ersten Phase darstellt und der Referenzwert der dritten Phase 0,95 bis 1,15 des täglichen Mittelwerts der zweiten Phase darstellt, wobei der Referenzwert der dritten Phase gegebenenfalls, im Zusammenhang mit einer anhaltenden Gewichtsänderung des Individuums, empirisch einstellbar ist.

13. Verfahren nach einem der Ansprüche 10 bis 12, **dadurch gekennzeichnet, dass** die zweite Phase mit dem Erreichen eines gewünschten Gewichts des Individuums endet.

14. Verfahren nach einem der Ansprüche 10 bis 12, **dadurch gekennzeichnet, dass** für spezifische Diätprogramme neben dem totalen Kalorienwert der aufgenommenen Nahrung, durch Erweiterung der gespeicherten Kalorienwerten der einzelnen Nahrungsmittel durch zusätzliche Speicherung des Gehaltes an Eiweiss oder Proteine, Kohlehydrate, Fett oder andere aus medizinischen Gründen zu begrenzende Nahrungsstoffe gemessen und dadurch in den gewünschten Grenzen gehalten werden können.

## Claims

1. Electronic scales for the efficient monitoring of the number of calories consumed, comprising at least one weight sensor or force sensor, a data processing unit with memory means, a control and input keypad and a display device,
said scales (1) having an active bearing surface (5) which is functionally connected to the weight or force sensor(s) and which is adapted to hold a plate or similar container,
the data processing unit (2) being provided with a program which calculates the calorie equivalence value for any dish, in the form of liquid and/or solid food, based on the weighed amount of food and the weight-related calorie content of said food which has been stored or input manually, adds the latter to the calorie equivalence values of the other dishes of a meal and calculates the total value of the calories of foods consumed daily, and stores, displays and/or prints out this value,
the overall structure of the scales (1) being essentially flat and thin,
the memory means (2') being suitable for storing preprogrammed target values which are calculated from the data inputs and measurements of a calibration period by the data processing unit (2) and are used as comparison or reference values during the period of calorie monitoring,
said scales (1) being **characterised in that**
- they include at least one second active bearing surface (6) which is separate from the first bearing surface (5), provided with a separate weight or force sensor(s) and is functionally connected to the latter and is suitable for holding drinking vessels or containers, such as e.g. glasses or cups, wherein at least one second measurement signal is supplied by the latter,
- they thus comprise bearing surface (5) for the plate and a bearing surface (6) for a drinking vessel,
they contain blocking means which make it possible to rigidly lock the bearing surface (5) for the plate and if necessary the bearing surface (6) of the drinking vessel, outside of the measuring and calculation phase, in a given position and/or block and prevent any data transfer as measuring signals to the data processing unit (2).

2. Electronic scales according to claim 1, **characterised in that** the bearing surface (5) and the weight or force sensors assigned thereto are designed such that plates of different sizes and shapes can be weighed accurately, and **in that** the program includes a routine which checks during each weight measurement phase that each weight or force sensor is loaded and delivers a correct measurement signal contributing to the measurement result.

3. Electronic scales according to claim 1 or 2, **characterised in that** the programming and control keypad (3) and the display device (4) consisting of liquid crystal diodes for example are pushed to the side, and thus can be seen or reached even when a plate and/or a drinking vessel is present.

4. Electronic scales according to claim 3, **characterised in that** they comprise two flat casings (1') which are hinged to one another via a hinge part (7), and the scales can thus be folded up when not in use and/or for transport.

5. Electronic scales according to any of claims 1 to 4, **characterised in that** the memory means (2') contain a programmable, completable and queriable calorie equivalence table which can be completed and retrieved by inputting data via the keypad (3), wherein the query can be performed by inputting only some of the letters of the name of the relevant dish/food and the queried value is used automatically by the data processing unit (2) as a multiplication factor for calculating the calorie value of a weighed dish/food.

6. Electronic scales according to claim 5, **characterised in that** the retrievable calorie equivalence table, for individuals who are suffering from a disease and thus need to consume specifically on a daily basis at least or at most values of an element or molecule, contains the specific content of the critical proportions of the given food list and its daily total is also calculated.

7. Electronic scales according to any of claims 1 to 6, **characterised in that** the program, via a corresponding control by the keypad (3), measures and stores the empty weight of the plate and/or the drinking vessel and afterwards calculates and stores the calorie value of each dish/food of a meal placed thereon, the total calorie value of each meal and the cumulative calorie value of the different meals of a day, wherein also the plus/minus difference from a daily calorie target value which has been calculated or input beforehand is calculated and displayed.

8. Electronic scales according to any of claims 1 to 7, **characterised in that** the stored values, possibly supplemented by the date and/or time and/or by data entered manually via the keypad (3), are transmitted by corresponding integrated or separate connected means to a data medium, such as e.g. a sheet of paper, an electronic data card, a solid state storage or the like.

9. Electronic scales according to any of claims 1 to 8, **characterised in that** they also comprise means for the visual or audible reproduction of messages, in particular results, comments, warnings, deviations from the stored diet program and the like.

10. Method for monitoring the food consumption of an individual within the framework of a diet and using electronic scales according to any of claims 1 to 9, which comprises a first active bearing surface (5) for holding plate or similar container and at least one second separate active bearing surface (6) for holding a drinking vessel or container, wherein the method in terms of time comprises three successive phases, specifically a first calibration period of limited duration, a subsequent effective diet period and finally a long-term stabilisation period, during all three periods the daily calorie equivalence values of the foods consumed being computed by means of the electronic scales (1), by weighing the individual foods of each meal, calculating the corresponding calories and adding up the latter for each day of these three periods, wherein the first bearing surface (5) delivers a first measurement signal and the second bearing surface (6) delivers a second measurement signals, wherein the scales (1) calculate and save from the input data and measurements of the calibration period the daily target calories, which are used as comparison or reference values during the two following periods, wherein the scales (1) are used during the meals as plate mats, after locking in a specific position and locking any transfer of data as measurement signals to the data processing unit (2), wherein in the third period or stabilisation period the efficiency or the correctness of the number of calories consumed daily is monitored by regularly weighing the body weight, and if necessary empirically corrected to maintain the desired body weight.

11. Method according to claim 10, **characterised in that** the values relating to the daily calorie intake of the individual during the first phase are used as the basis for calculating reference values for the second and third phase, wherein in the second and third phase a deviation of the effective daily calorie intake value from the corresponding pre-calculated reference value is given, displayed and/or reported.

12. Method according to claim 10 or 11, **characterised in that** the reference value of the second phase is 0.75 to 0.90 of the daily average of the first phase and the reference value of the third phase is 0.95 to 1.15 of the daily average of the second phase, the reference value of the third phase being empirically adjustable in conjunction with the prevailing weight change of the individual.

13. Method according to any of claims 10 to 12, **characterised in that** the second phase ends when the desired weight of the individual is reached.

14. Method according to any of claims 10 to 12, **characterised in that** for specific diet programs, in addition to the total calorie value of the food consumed, by expanding the stored calorie values of the individual foods, by additional storage of the content of proteins, carbohydrates, fat or other foods which are to be limited for medical reasons are measurable and can thereby be kept within the desired limits.

## Revendications

1. Balance électronique dévolue au contrôle efficace des quantités de calories ingérées, comprenant au moins un capteur de poids ou de forces, une unité de traitement de données dotée de moyens de mémorisation, un clavier de commande et de saisie, et un dispositif d'affichage,
cette balance (1) étant munie d'une surface de contact opérante (5), appropriée pour recevoir une assiette ou un réceptacle similaire et connectée fonctionnellement au(x) capteur(s) de poids ou de forces,
l'unité (2) de traitement de données étant pourvue d'un programme qui, pour chaque mets se présentant comme un aliment liquide et/ou solide, sur la base de la quantité de mets pesée et de la teneur en calories rapportée au poids de ce mets, mémorisée ou saisie manuellement, calcule la valeur d'équivalent calorique, additionne cette dernière aux valeurs d'équivalent calorique des autres mets d'un repas, et calcule une valeur totale des calories des mets quotidiennement ingérés, puis mémorise, affiche et/ou imprime cette valeur,
l'agencement structurel d'ensemble de ladite balance (1) étant substantiellement plat et mince,
les moyens de mémorisation (2') étant appropriés pour renfermer des valeurs de consigne préprogrammées qui sont calculées par ladite unité (2) de traitement de données, à partir des saisies de données et des mesures d'une période d'étalonnage, et servent de valeurs de comparaison ou de référence durant la période de contrôle calorique,
balance (1) **caractérisée par le fait :**
- **qu'**elle inclut au moins une seconde surface de contact opérante (6) qui est distincte de la première surface de contact (5), est équipée d'un (de) propre(s) capteur(s) de poids ou de forces au(x)quel(s) elle est fonctionnellement connectée, est appropriée pour recevoir des récipients ou réceptacles à boissons tels que des verres ou des tasses, par exemple, et délivre au moins un second signal de mesure,
- **qu'**elle comporte, de la sorte, une surface de contact (5) dédiée à l'assiette et une surface de contact (6) dédiée à un récipient à boissons,
- **qu'**elle inclut des moyens de blocage qui permettent de verrouiller rigidement dans une position donnée, en dehors d'une phase de mesure et de calcul, ladite surface de contact (5) dédiée à l'assiette et, le cas échéant, ladite surface de contact (6) dédiée au récipient à boissons, et/ou qui bloquent et empêchent un quelconque transfert, à ladite unité (2) de traitement de données, de données se présentant comme des signaux de mesure.

2. Balance électronique selon la revendication 1, **caractérisée par le fait que** la surface de contact (5), et les capteurs de poids ou de forces qui lui sont associés, sont conçus de façon telle que des assiettes de tailles et de formes différentes puissent être pesées avec précision ; et **par le fait que** le programme renferme une routine qui vérifie que chaque capteur de poids ou de forces est chargé, lors de chaque phase de mesure pondérale, et délivre un signal de mesure correct concourant au résultat de la mesure.

3. Balance électronique selon la revendication 1 ou 2, **caractérisée par le fait que** le clavier (3) de programmation et de commande, ainsi que le dispositif d'affichage (4) consistant, par exemple, en des diodes à cristaux liquides, sont agencés avec décalage latéral et peuvent, de la sorte, être respectivement observés ou atteints même en présence d'une assiette et/ou d'un récipient à boissons.

4. Balance électronique selon la revendication 3, **caractérisée par le fait qu'**elle est constituée de deux enveloppes (1') de forme aplatie, reliées l'une à l'autre de manière articulée par l'intermédiaire d'une partie (7) formant charnière, ladite balance pouvant ainsi être repliée sur elle-même en cas de non-utilisation, et/ou en vue du transport.

5. Balance électronique selon l'une des revendications 1 à 4, **caractérisée par le fait que** les moyens de mémorisation (2') renferment un tableau d'équivalents caloriques programmable, évolutif et consultable, qui peut être complété et interrogé par saisie de données à l'aide du clavier (3), sachant que l'interrogation peut être effectuée en saisissant uniquement une partie des lettres du nom du mets/de l'aliment concerné, et que la valeur respectivement interrogée est utilisée automatiquement, par l'unité (2) de traitement de données, en tant que facteur de multiplication affecté au calcul de la valeur calorique d'un mets/d'un aliment pesé.

6. Balance électronique selon la revendication 5, **caractérisée par le fait que**, pour des personnes atteintes d'une maladie et devant spécifiquement, en conséquence, ingérer quotidiennement des valeurs minimales ou maximales d'un élément ou d'une molécule, le tableau d'équivalents caloriques consultable renferme la teneur spécifique des parts critiques de la liste de mets préétablie ; et **par le fait que** le total quotidien de ladite teneur est pareillement calculé.

7. Balance électronique selon l'une des revendications 1 à 6, **caractérisée par le fait que**, sur commande correspondante lancée à l'aide du clavier (3), le programme mesure et mémorise le poids à vide de l'assiette et/ou du récipient à boissons, et calcule et mémorise ensuite la valeur calorique de chaque mets/aliment déposé, constitutif d'un repas, la valeur calorique totale de chaque repas et la valeur calorique cumulée des différents repas d'une journée, également avec calcul et affichage éventuels de la différence en plus/en moins par rapport à une valeur calorique de consigne quotidienne, préalablement saisie ou calculée au préalable.

8. Balance électronique selon l'une des revendications 1 à 7, **caractérisée par le fait que** les valeurs mémorisées, complétées le cas échéant par une indication de date et/ou de temps et/ou par des données saisies manuellement à l'aide du clavier (3), sont transférées, par des moyens correspondants, intégrés ou connectés distinctement, à un support de données se présentant, par exemple, comme une feuille de papier, une carte électronique de données, une mémoire à corps solide ou un élément similaire.

9. Balance électronique selon l'une des revendications 1 à 8, **caractérisée par le fait qu'**elle est également pourvue de moyens de restitution visuelle ou sonore d'avertissements, en particulier de notifications de résultats, de commentaires, d'alertes, d'écarts vis-à-vis du programme de régime mémorisé, et considérations analogues.

10. Procédé de contrôle de la prise d'aliments par un individu dans le cadre d'un régime, avec utilisation d'une balance électronique conforme à l'une des revendications 1 à 9, munie d'une première surface de contact opérante (5) conçue pour recevoir une assiette ou un réceptacle similaire, et d'au moins une seconde surface de contact opérante (6) distincte, conçue pour recevoir un récipient ou un réceptacle à boissons,
ledit procédé étant composé de trois phases se succédant mutuellement dans le temps, à savoir d'une première période d'étalonnage à délimitation temporelle, d'une période successive de régime effectif et, pour finir, d'une période de stabilisation de longue durée,
sachant que, durant l'intégralité des trois périodes, les valeurs quotidiennes d'équivalent calorique des mets ingérés sont calculées, au moyen de ladite balance électronique (1), par pesage des mets individuels de chaque repas, par calcul des calories correspondantes et par sommation de ces dernières pour chaque journée de ces trois périodes,
sachant que ladite première surface de contact (5) délivre un premier signal de mesure et que ladite seconde surface de contact (6) délivre un second signal de mesure, ladite balance (1) calculant et mémorisant, à partir des saisies de données et des mesures de ladite période d'étalonnage, des valeurs de consigne caloriques quotidiennes servant de valeurs de comparaison ou de référence durant les deux périodes suivantes,
sachant que ladite balance (1) sert de soucoupe au cours des repas, après verrouillage dans une position donnée et après blocage d'un quelconque transfert, à l'unité (2) de traitement de données, de données se présentant comme des signaux de mesure,
sachant que, lors de la troisième période ou période de stabilisation, l'effectivité ou, respectivement, la pertinence de la quantité de calories quotidiennement ingérée est contrôlée par pesage régulier de la masse corporelle et est corrigée empiriquement, si nécessaire, afin d'entretenir la masse corporelle souhaitée.

11. Procédé selon la revendication 10, **caractérisé par le fait que** les valeurs relatives à une ingestion quotidienne de calories par l'individu, durant la première phase, servent de base de calcul de valeurs de référence affectées aux deuxième et troisième phases, sachant que, durant lesdites deuxième et troisième phases, un écart de la valeur effective d'ingestion quotidienne de calories, vis-à-vis de la valeur de référence correspondante calculée au préalable, est indiqué, affiché et/ou notifié.

12. Procédé selon la revendication 10 ou 11, **caractérisé par le fait que** la valeur de référence de la deuxième phase représente de 0,75 à 0,90 de la valeur moyenne quotidienne de la première phase, et la valeur de référence de la troisième phase représente de 0,95 à 1,15 de la valeur moyenne quotidienne de ladite deuxième phase, ladite valeur de référence de la troisième phase pouvant être, le cas échant, réglée empiriquement en rapport avec une variation durable du poids de l'individu.

13. Procédé selon l'une des revendications 10 à 12, **caractérisé par le fait que** la deuxième phase s'achève lorsqu'un poids souhaité de l'individu est atteint.

14. Procédé selon l'une des revendications 10 à 12, **caractérisé par le fait que** pour des programmes de régime spécifiques, outre la valeur calorique totale de l'aliment ingéré, des hydrates de carbone, des lipides ou d'autres substances nutritives devant être restreintes pour raisons médicales sont mesuré(e)s et peuvent, de la sorte, être maintenu(e)s dans les limites souhaitées en extrapolant les valeurs caloriques mémorisées des nutriments individuels, par mémorisation supplémentaire de la teneur en albumine ou en protéines.
